# EUROPEAN PATENT SPECIFICATION

(11) **EP 3 405 204 B1**
(45) Date of publication and mention of the grant of the patent: **25.06.2025**
(21) Application number: 16885394.3
(22) Date of filing: 01.11.2016
(51) Int. Cl.: A61K 35/51, C12N 5/02, C12M 3/00, C12N 5/073, C12N 5/0775

(54) **COMPOSITION AND METHODS OF USING UMBILICAL CORD LINING STEM CELLS**
ZUSAMMENSETZUNG UND VERFAHREN ZUR VERWENDUNG VON NABELSCHNURSTAMMZELLEN
COMPOSITION ET PROCÉDÉS D'UTILISATION DE CELLULES SOUCHES DE LA PAROI INTÉRIEURE DU CORDON OMBILICAL

(30) Priority: 26.08.2016 US 201615248629
(43) Date of publication of application: 28.11.2018
(60) Divisional application: 25177561.5
(73) Proprietor: RESTEM LLC, Miami, FL 33145 (US)
(72) Inventor: GONZALEZ, Rafael, Yorba Linda, CA 92887 (US)
(74) Representative: Schlich
(86) International application number: PCT/US2016/059937
(87) International publication number: WO 2017/123312

(56) References cited:
- EP-A1- 2 597 149
- WO-A1-2013/093878
- US-A1- 2006 153 815
- US-A1- 2006 153 815
- US-A1- 2012 251 489
- US-A1- 2013 065 302
- US-A1- 2013 065 302
- US-A1- 2014 045 263
- US-A1- 2015 250 823
- MARIA TERESA CONCONI: "Phenotype and Differentiation Potential of Stromal Populations Obtained from Various Zones of Human Umbilical Cord: An Overview", THE OPEN TISSUE ENGINEERING AND REGENERATIVE MEDICINE JOURNAL, vol. 4, no. 1, 30 December 2011 (2011-12-30), pages 6 - 20, XP055062745, ISSN: 1875-0435, DOI: 10.2174/1875043501104010006
- JESCHKE M G ET AL: "Umbilical cord lining membrane and wharton's jelly-derived mesenchymal stem cells: The similarities and differences", OPEN TISSUE ENGINEERING AND REGENERATIVE MEDICINE JOURNAL 2011 BENTHAM SCIENCE PUBLISHERS B.V. NLD,, vol. 4, no. SPEC. ISSUE 1, 1 January 2011 (2011-01-01), pages 21 - 27, XP002696906, ISSN: 1875-0435, DOI: 10.2174/1875043501104010021
- CHONGYANG SHEN ET AL: "Conditioned medium from umbilical cord mesenchymal stem cells induces migration and angiogenesis", MOLECULAR MEDICINE REPORTS, vol. 12, no. 1, 4 March 2015 (2015-03-04), GR, pages 20 - 30, XP055652950, ISSN: 1791-2997, DOI: 10.3892/mmr.2015.3409

## Description

### FIELD OF INVENTION

The invention relates to umbilical cord stem cells (UCSCs) and their therapeutic uses. UCSCs are replacing bone marrow derived stem cells (BMSCs) as the standard of care for many blood diseases because ULSCs have greater potency and are more easily isolated. The present invention relates to UCSCs derived from the lining of the umbilical cord (ULSCs) and their use in the production of valuable glycosaminoglycans (GAGs).

### BACKGROUND OF THE INVENTION

In our bodies there is constant loss and replacement of cells in the physiological process known as homeostasis. Homeostasis ensures that when we lose cells to every day wear and tear they are replaced by newly born cells derived from stem cells. Some tissues have stem cells that supply new cells at a fast rate like skin while others like the brain have slowly dividing stem cells capable of only limited cellular replacement. As we age replacement rates in all our tissues decrease because there are fewer surviving stem cells. With less cellular replacement eventually organs are inadequately repaired and fail.

It was discovered in the 1950's that Hematopoietic Stem Cells (HSCs) harvested from adult bone marrow can be used to treat leukemia after radiation or chemotherapy (Mayani 2003, Copelan 2006). Transplanted HSCs seek out bone marrow and produce new blood including both myeloid and lymphoid cells. Today bone derived HSC transplantation is effective for treatment of leukemias, severe aplastic anemia, lymphomas, multiple myeloma and immune deficiency disorders among others. There are now over 50,000 transplantations per year worldwide (Von Ah et al. 2015). Bone marrow blood banking and human leukocyte antigen (HLA) matching enables patients to draw upon a larger donor pool thereby further expanding the effectiveness of HSC-transplantation therapy.

Adult bone marrow contains another important type of stem cell known as Mesencyhymal Stem Cells (MSCs). Although first isolated from bone marrow MSCs represent only a small fraction of total bone marrow cells. Fortunately MSCs are more prevalent in other tissues including fat, teeth and umbilical cords. MSCs are multipotent giving rise to a broad range of cell types but primarily form bone and connective tissue cells (Gonzalez et al. 2015, Pittenger et al. 1999). MSCs are useful in treating joint and connective tissue injuries where repair is often limited due to a lack of blood supply. When MSCs are transplanted into joints and other connective tissues they recruit blood vessels into the vicinity thereby enhancing repair.

It is now appreciated that MSCs also enhance injury repair by significantly reducing inflammation (Gonzalez et al. 2015). MSCs reduce inflammation in several ways. MSCs avoid provoking an immune response by expressing neither class I nor class II major histocompatibility proteins (MHC) (De Miguel et al. 2012). MSCs also express inflammation reducing proteins including interleukin (IL) 4, IL-6, IL-10, prostaglandin E2 (PGE2), HLA-G and transforming growth factor (TGF) β1 (DeMiguel et al. 2012, Gonzalez et al. 2015, Aggarwal 2005). The presence of these and other factors secreted by MSCs induces nearby cells to also produce antiinflammatory factors and to reduce expression of pro-inflammatory factors such as IL-6, and tumor necrosis factor alpha (TNFα) (Gonzalez 2015 et al.).

An increasingly prevalent source of both HSCs and MSCs is human umbilical cords. Umbilical cord derived stem cells (UCSCs) are an attractive alternative to BMSCs because they are more potent, having greater proliferation and differentiation potential due to their fetal origin. UCSCs are also more cost effective to harvest compared to BMSCs. The umbilical cord in a full term baby averages 500 millimeters in length and about 20 millimeters in diameter. The outer most cord tissue is known as the amniotic epithelium. It forms a tube which contains collagen fibers, fibroblast cells, and Wharton's jelly, (a gelatinous substance made largely from mucopolysaccharides and extracellular matrix) as well as two arteries, a vein and allantois duct. Both HSCs and MSCs can be extracted from these structures having been found in cord blood, the vein, arteries, the amniotic epithelia, endothelium and Wharton's jelly (Subramanian et al. 2013).

The present invention relates to umbilical cord lining derived MSCs which are isolated from the amniotic epithelia including but not limited to ULSCs isolated by the method described in US Patent 8,778,679.

### RELATED ART

US 8778679**-** Umbilical Cord Lining Stem Cells And Methods And Material For Isolating And Culturing Same.

US 20120142102 A1-Isolation of human umbilical cord blood-derived mesenchymal stem cells-

US 20120021509 A1-Isolating method for umbilical cord blood-derived pluripotent stem cells expressing znf281.

EP 2079829 A2-Differentiation of stem cells from umbilical cord matrix into hepatocyte lineage cells.

US 20080292597 A1**-** Umbilical Cord Stem Cell Composition & Method of Treating Neurological Diseases.

US 9017657 B2 -Islet cell cluster produced from human umbilical cord mesenchymal stem cells. The invention also provides a method for using a stem cell conditioned medium to culture animal cells.

US 20130157365 A1-Buensuceso-Induced pluripotent stem cells from human umbilical cord tissue-derived cells

WO 2014013255 A1**-** Erythroid production - This invention provides method and media suitable for inducing and supporting the differentiation of stem cells into erythroid cells.

WO 2009137629 A2-Methods for producing enucleated erythroid cells derived from pluripotent stem cells.

US 6217888 B1**-** Methods of Regulating Skin Appearance with Vitamin B3 compound.

### REFERENCES CITED

Gonzalez, R. et al. Stem Cells Targeting Inflammation as Potential Anti-aging Strategies and Therapies. Cell and Tissue Transplantation and Therapy. Libertas Academic. 2015:7.
DeMiguel, M.P. et al. Immunosuppressive properties of mesenchymal stem cells: advances and applications. Curr Mol Med. 2012; 12:574-591.
Calabro, A. et al. Fluorophore-assisted carbohydrate electrophoresis (FACE) of glycosaminoglycans. Journal of the Osteoarthritis Research Society International. 2001:9, Supp A: 16-22.
Calabro, A. et al. Microanalysis of enzyme digests of hyaluronan and chondroitin/dermatan sulfate by flurophore-assisted carbohydrate electrophoresis (FACE). Glycobiology: vol 10 no. 3, p. 273-281 (2000).
Cantinieaux, D. et al. Conditioned Medium from Bone Marrow-Derived Mesenchymal Stem Cells Improves Recovery after Spinal Cord Injury in Rats: An Original Strategy to Avoid Cell Transplantation. PLOS One. August 2013, Vol 8, Iss 8.
Tullberg-Reinert, H. et al. In situ measurement of collagen synthesis by human bone cells with a Sirius Red-based colorimetric microassay: Effects of transforming growth factor beta 2 and ascorbic acid 2-phosphate. Histochem Cell Biol (1999) 112:271-276.
Copelan, E.A. Hematopoietic Stem-Cell Transplantation. The New England Journal of Medicine. 2006: 354: 1813-26.
Ariff, B. et al. The Therapeutic Potential, Challenges and Future Clinical Directions of Stem Cells from the Wharton's Jelly of the Human Umbilical Cord. Stem Cell Reviews and Reports. April 2013, Volume 9, Issue 2, pp 226-240
Broxmeyer, H. et al. Human umbilical cord blood as a potential source of transplantable hematopoietic stem/progenitor cells. PNAS. 1989 May; 86(10):3828-32.
Mayani, H. A glance into somatic stem cell biology: basic principles, new concepts, and clinical relevance. Arch Med Res. 2003, 34:3-15.
Thomson, J. et al. Blastocysts Embryonic Stem Cell Lines Derived from Human. 1998. Science 282 (5391): 1145-1147.
Chambers, I. et al. Functional expression cloning of Nanog, a pluripotency sustaining factor in embryonic stem cells 2003, Cell, vol 113, no. 5, pp. 643-55.
Lu, L.L. et al. Isolation And Characterization Of Human Umbilical Cord Mesenchymal Stem Cells With Hematopoiesis-Supportive Function And Other Potentials Haematologica January 2006 91: 1017-1026.
Chen, G. et al. Human Umbilical Cord-Derived Mesenchymal Stem Cells Do Not Undergo Malignant Transformation during LongTerm Culturing in Serum-Free Medium, PLOS, June 2014, Volume 9, Issue 6, e98565.
Von Ah, D. et al. The Caregiver's Role Across the Bone Marrow Transplantation Trajectory. Cancer Nursing, Vol. 0, No. 0, 2015.
Subramanian, A. et al. Comparative Characterization of Cells from the Various Compartments of the Human Umbilical Cord Shows that the Wharton's Jelly Compartment Provides the Best Source of Clinically Utilizable Mesenchymal Stem Cells. PLOS One. June 10, 2015.
Frisbie, D.D. et al. Clinical update on the use of mesenchymal stem cells in equine orthopaedics. Equine Veterinary Journal, 2010, 42: 86-89.
Lee, S.J. et al. High-resolution donor-recipient HLA matching contributes to the success of unrelated donor marrow transplantation. Blood Dec 2007, 110 (13) 4576-4583.
Halme, D.G. et al. FDA Regulation of Stem-Cell-Based Therapies. N Engl J Med 2006; 355:1730-1735,October 19, 2006.
Civin, C.I., et al. Antigenic analysis of hematopoiesis. III. A hematopoietic progenitor cell surface antigen defined by a monoclonal antibody raised against KG-1a cells. J Immunol 1984;133:157- 165.
Dominici M., et al. Minimal criteria for defining multipotent mesenchymal stromal cells. The International Society for Cellular Therapy position statement. Cytotherapy 8 (4): 315-317. 2006.
Pittenger M.F., et al. Multilineage potential of adult human mesenchymal stem cells. Science, 1999, Apr 2; 284(5411):143-7.
Kern, S. et al. Comparative Analysis of Mesenchymal Stem Cells from Bone Marrow, Umbilical Cord Blood, or Adipose Tissue. STEM CELLS, 24: 1294-1301. 2006.
Shen, C. et al. Conditioned medium from umbilical cord mesenchymal stem cells induces migration and angiogenesis. Molecular Medicine Reports 12.1 (2015): 20-30.
Guan, L. et al. Therapeutic efficacy of umbilical cord-derived mesenchymal stem cells in patients with type 2 diabetes". Experimental and Therapeutic Medicine 9.5 (2015): 1623-1630. Tordeschi, M.R. et al. Transplanted Umbilical Cord Mesenchymal Stem Cells Modify the In Vivo Microenvironment Enhancing Angiogenesis and Leading to Bone Regeneration. Stem cells and development. Stem Cells Dev. 2015. Jul 1;24(13):1570-81.
Wang K.X et al. The Effects of Secretion Factors from Umbilical Cord Derived Mesenchymal Stem Cells on Osteogenic Differentiation of Mesenchymal Stem Cells. PLoS ONE 10(3): 2015.
Kyurhchiev, D. et al. Secretion of immunoregulatory cytokines by mesenchymal stem cells. World Journal of Stem Cells. Nov 26; 6(5): 552-570. 2014.

### SUMMARY OF THE INVENTION

The present invention provides methods of using ULSCs.

Non-limiting examples of applications of ULSCs according to the methods herein include production of glycosaminoglycans (GAGs).

In a particular embodiment, Embodiment 1, the invention provides a method of using umbilical cord lining stem cells (ULSCs) to produce glycosaminoglycans, the method comprising:
a. Propagating ULSCs in a growth media or conditioning media;
b. Filtering and concentrating the growth media or conditioning media from step a.;
c. Treating the filtered and concentrated growth media or conditioning media from step b. with proteases and nucleases to remove protein and nucleic acid;
d. Treating the treated growth media or conditioning media from step c. with a detergent and an alcohol to remove lipids;
e. Harvesting glycosaminoglycans from the treated growth media or conditioning media from step d.

In another embodiment, Embodiment 2, the invention may provide a method according to Embodiment 1 wherein the glycosaminoglycans are selected from one or more of hyaluronic acid and chondroitin sulfate.

In another embodiment, Embodiment 3, the invention may provide a method according to Embodiment 2 wherein the hyaluronic acid has a molecular weight of at least 2500 kD.

In a final embodiment, Embodiment 4, the invention may provide a method of any one of Embodiments 1 to 3 comprising the further step of purifying glycosaminoglycans.

The present invention achieves its objects by methods of propagating ULSCs that produce accumulations of glycosaminoglycans (GAGs) in conditioned media and within the cell layer that may be harvested for therapeutic uses. The manners in which the invention achieves its objects and other objects which are inherent in the invention will become more readily apparent when reference is made to the accompanying drawings.

### BRIEF DESCRIPTION OF THE DRAWINGS

**FIGURE 1****.** contains histograms of cytokines and chemokines in ULSC and BMSC conditioned media assayed by a quantitative multiplexed immunoassay system (MAPs).
**FIGURE 2****.** contains histograms of growth factor release in ULSC and BMSC conditioned media assayed by Enzyme Linked Immunosorbent Assay (ELISA).
**FIGURE 3****.** contains fluorophore-assisted carbohydrate electrophoresis (FACE) analysis demonstrating size and quantity of Hyaluronic Acid (HA) and Chrondroitin Sulfate (CS) in ULSC and BMSC conditioned media.
**FIGURE 4****.** contains an image of a hyaluronan size analysis by agarose gel electrophoresis.
**FIGURE 5****.** contains an image of a hyase digest size analysis by agarose gel electrophoresis.
**FIGURE 6****.** contains a histogram depicting levels of HA and CS in both conditioned media samples and the cells that produced said media.
**FIGURE 7****.** contains a data table and absorbance curve of Collagen in ULSC conditioned media assayed by dilution and absorbance analysis.

### DETAILED DESCRIPTION OF THE INVENTION

Various embodiments of the invention are described in detail and may be further illustrated by the provided examples. As used in the description herein and throughout the claims that follow, the meaning of "a," "an," and "the" includes the plural reference unless the context clearly dictates otherwise. Also, as used in the description herein, the meaning of "in" includes "in" and "on" unless the context clearly dictates otherwise.

Throughout this specification and claims, the word "comprise," or variations such as "comprises" or "comprising" will be understood to imply the inclusion of a stated integer or group of integers but not the exclusion of any other integer or group of integers.

Terms used in this specification generally have their ordinary meanings in the art, within the context of the invention, and in the specific context where each term is used. Certain terms that are used to describe the invention are discussed below, or elsewhere in the specification, to provide additional guidance to the practitioner in describing the compositions and methods of the invention and how to make and use them. For convenience, certain terms may be highlighted for example using italics and/or quotation marks. The use of highlighting has no influence on the scope and meaning of a term; the scope and meaning of a term is the same, in the same context, whether or not it is highlighted. It will be appreciated that the same thing can be said in more than one way. Consequently, alternative language and synonyms does not exclude the use of other synonyms. The use of examples anywhere in this specification, including examples of any terms discussed herein, is illustrative only, and in no way limits the scope of the invention so long as the data are processed, sampled, converted, or the like according to the invention without regard for any particular theory or scheme of action.

Further, whenever the name of a specific growth factor, cytokine, chemokine or extracellular matrix component is given, it is assumed to include all homologues, family members and splice variants of that factor which may also be present.

In all of the following examples, of which Example 3 represents a non-limiting preferred embodiment of the invention and Examples 1, 2 and 4 are not according to the invention but are present for illustration purposes only, pure ULSC cell lines obtained according to prior art methods (US 8778679) and BMSC lines are propagated based upon the following protocol. Culture vessels are seeded with cells at density of 1x10³ cells per cm² in Dulbecco's Modified Eagle Medium (DMEM) with low glucose and 15% fetal bovine serum (FBS), hereafter referred to as expansion media. Every 3 days the expansion media is replaced with fresh sterile expansion media. ULSCs are allowed to grow in a CO₂ incubator at 37°C until they reach 80-90% confluency. Once confluent the media is replaced with sterile conditioning media consisting of 98% Roswell Park Memorial Institute Medium (RPMI), 2% FBS, 1 milliliter (ml) 100x Penicillin and Streptomycin, 1 ml 100x Non Essential Amino Acids (NEAA), 1 ml 100x Modified Eagle Medium (MEM), 1ml Insulin, Transferrin, Selenium (ITS) supplement, 1mL 100x Glutamine supplement. Conditioned media is collected at regular intervals into a sterile polyethylene terephthalate (PET) container that is stored at -20°C and replaced with fresh sterile conditioning media. The process of collecting conditioned media by this method may be continued indefinitely.

Where applicable in the examples conditioned media may be purified and concentrated according to the following protocol. Collected conditioned media is thawed at 4°C. Collected lots are pooled and then filtered with a 0.22 micron (um) filter. Filtered conditioned media is added to sample filter centrifugation cup which is then added to the filtrate collection cup and the combination is placed in a balanced centrifuge. The centrifuge is spun at up to 3500xg until a desired concentration is obtained, typically between 50-90 minutes. Concentrated media may then be transferred from the filtrate collection cup for further application as desired.

### EXAMPLE 1: CYTOKINE AND CHEMOKINE ASSAY

Media from ULSCs, two sets from passage 3 and two sets from passage 6, in both expansion and conditioned media and four identical cultures of BMSCs were assayed for the presence of cytokine and chemokine proteins by quantitative multiplexed immunoassay analysis (MAPs). The cytokines and chemokines profiled included: GM-CSF, INF-γ, IL-3, IL-4, IL-6, IL-7, IL-8, IL-18, MIP-1β, MCP-1, TNF-α. The MAPs analysis is based upon a capture-sandwich wherein capture antibodies are attached to fluorescently encoded microspheres. After capture of antigen from a biological sample such as cell culture media the antigen is detected and quantified using specific detection antibodies coupled to a fluorescent probe. Results obtained from this analysis were given as weight per volume of each analyte. The results were then normalized per number of cells from the corresponding well.

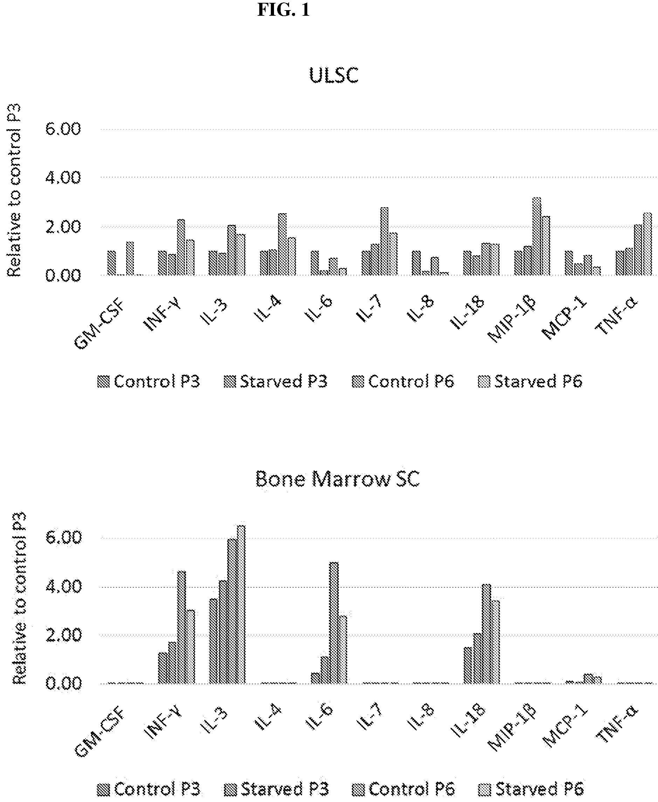

The results in FIG. 1 demonstrate that ULSCs express and secrete considerably more GM-CSF, IL-4, IL-7, IL-8, MIP-1β, MCP-1, and TNF-α than BMSCs. ULSCs express all the factors made by BMSCs and at least seven more factors not found at significant levels in BMSCs, These results are indicative but not exhaustive of the cytokines and chemokines which may be present in ULSCs conditioned media and cell culture tissue. Other cytokines which may be present include those from all four cytokine families: the four α-helix bundle family (the IL-2 subfamily, the interferon subfamily, the IL-10 subfamily), the IL-1 family (primarily IL-1 and IL-18), the IL-17 family and the cysteine-knot family (transforming growth factor beta superfamily including TGF-β1, TGF-β2, TGF-β3). Other chemokines which may be present include those from all four classes including C chemokines (XCL1 and XCL2), CC chemokines (CCL1-CCL28), CXC chemokines (CXCL1-CXCL17), CX3C chemokines (CX3CL1).

### EXAMPLE 2: HUMAN GROWTH FACTOR ASSAY

Conditioned media obtained from ULSCs and BMSCs was assayed for the presence of VEGF and SCF proteins by an Enzyme Linked Immunosorbent Assay (ELISA). Conditioned media was collected from a total of 12 cultures, two sets of ULSCs and two sets of BMSCs at 3, 6, and 9 days. Controls tested included 2% FBS media, 0% FBS media, 0% media plus vitamin B3, ULSCs in chondro media, and ULSCs in a transwell plate with a chondrocyte pellet. Concentrated conditioned media from each sample was added to each ELISA test strip well and incubated for one hour with gentle shaking. The ELISA strip was washed with assay buffer to stop the reaction and the optical density of each ELISA strip well was measured with a microplate reader at 450nm.

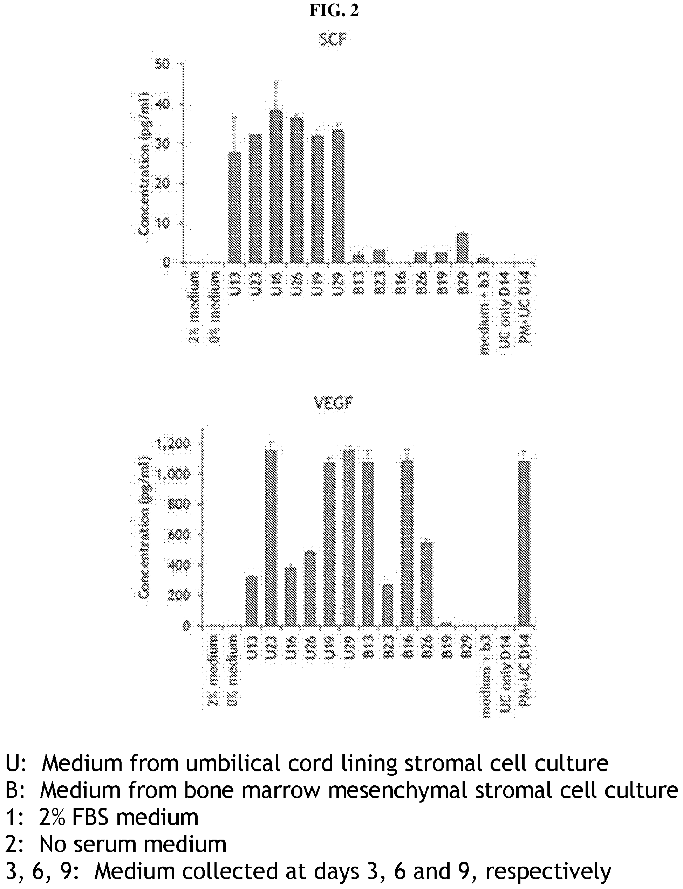

The results in FIG. 2 demonstrate that SCF is expressed and secreted at a considerably higher level in both sets of ULSCs at all time points compared to both sets of BMSCs. The results also show that both ULSCs and BMSCs express significant levels of VEGF but that after 9 days BMSCs secrete less VEGF than ULSCs. These non-limting examples are indicative but not exhaustive of the growth factors which may be present in ULSCs conditioned media and cell culture tissue. Other growth factors which may be present include VEGF family proteins, Epidermal Growth Factor (EGF) family proteins, Fibroblast Growth Factor (FGF) family proteins, Transforming Growth Factor beta (TGF-β) family proteins, Angiopoietin family proteins, Brain Derived Neurotrophic factor family proteins.

### EXAMPLE 3: HYALURONIC ACID AND CHONDROITIN SULFATE ASSAYS

Media was collected from both ULSCs and BMSCs either daily or every third day from cell cultures propagated in either growth media or conditioning media. The cell layers in each well were also analyzed. The filtered and concentrated media is treated with proteases and nucleases to remove protein and nucleic acid, then treated with detergent and alcohol to remove lipids, leaving a mixture enriched with complex carbohydrates including glycosaminoglycans (GAGs). The GAGs mixture was then treated with a hyaluronidase enzyme which cleaves the GAGs into their constituent parts. The hyaluronic acid fragments were then separated by either polyacrylamide gel electrophoresis (PAGE) or agarose gel electrophoresis.

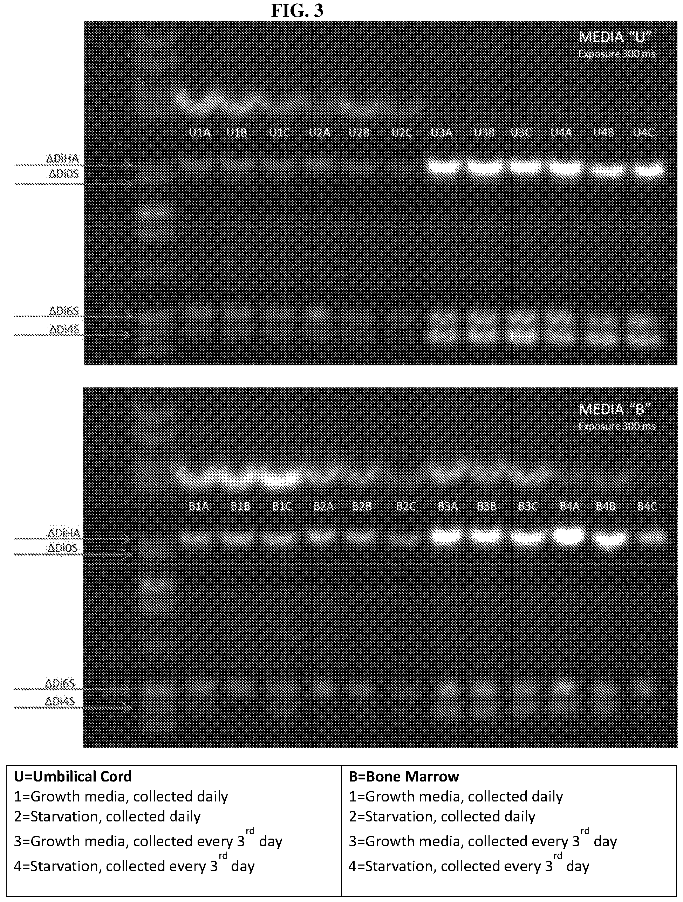

In FIG. 3, the cleaved GAGs were treated with mercuric ion, and then tagged by reductive amination giving an identical fluorescent signal for every free reducing group. The fluoro-tagged products were then separated by PAGE and scanned by CCD camera then analyzed with quantitative image analysis software. The results shown here demonstrate the presence of significant levels of HA and CS in the conditioned media of both ULSCs and BMSCs. These non-limiting examples are indicative but not exhaustive of the extracellular matrix constituents which may be present in ULSCs conditioned media and cell culture tissue. Other extracellular matrix constituents which may be present include: heparan sulfate, keratin sulfate, elastin fibers, fibronectins and laminins.

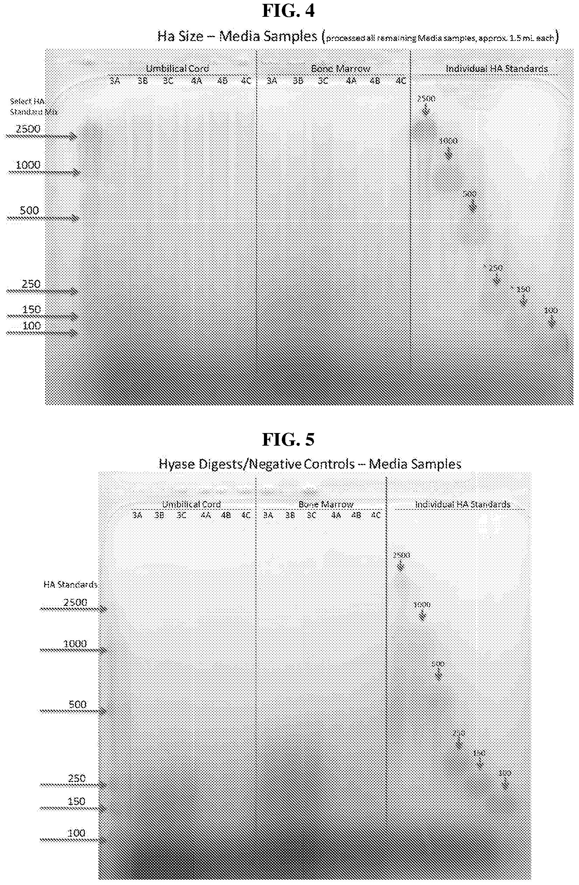

In FIG. 4 and FIG. 5, the cleaved GAGs were then separated on an agarose gel and stained for visualization prior to imaging. The results in FIG. 4 and FIG. 5 demonstrate that a considerable fraction of the HA present is in the media of both ULSC and BMSC cultures is high molecular weight up to at least 2500 kD. When further digested with Hyase (FIG.5), HA accumulates in low molecular weight fragments at the bottom of the gel.

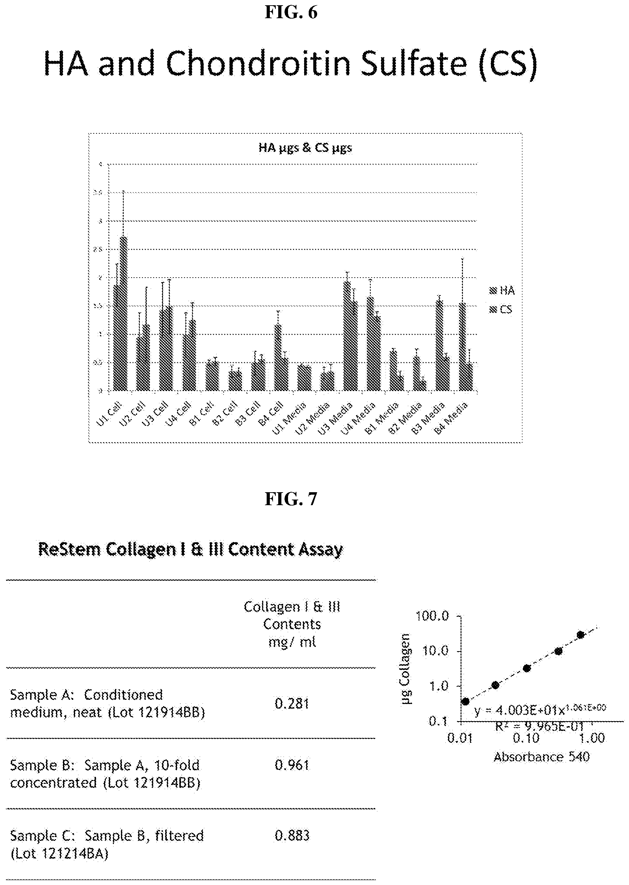

FIG. 6 provides a quantitation of the amounts of HA and CS present in both ULSC and BMSC cells and conditioned media. ULSC cells have increased quantities of both HA and CS compared to BMSCs and in conditioned media ULSCs produced more CS than BMSCs whereas HA levels were similar.

### EXAMPLE 4: COLLAGEN ASSAY

Collagen standards are prepared at 3⁰, 3⁻¹, 3⁻², 3⁻³ and 3⁻⁴ dilutions from 3 mg/ ml stock in phosphate buffered saline. Ten microliters of ULSC conditioned media is used to make Collagen samples in triplicate. Sample A is cell culture medium supplemented with 2 % fetal bovine serum and 1 % ITS conditioned for 2 days by confluent ULSCs. Sample B is sample A concentrated 10 fold by centrifugation filter. Sample C is sample B filtered through 3MM Whatman paper. The test samples are deposited in a well of a 96-well cluster, and air-dried. Sample A is tested as 3⁰, and 3⁻¹ dilutions; samples B and C are tested as 3⁰, 3⁻¹, 3⁻², and 3⁻³ dilutions. One hundred microliters Bouin's fluid were added to each well and incubated at 37 °C, for 1 hour, in a humidified enclosure. The Bouin's fluid was removed by aspiration, and the well was washed five times, each time with 200 µl deionized water, and air-dried. Seventy five microliters working Sirius Red solution were added to each well, and also to a set of 3 empty wells, serving as background controls, and incubated at 37 °C, for 1 hour, in a humidified enclosure. The dye solution was removed by aspiration, and the well was washed five times, each time with 400 µl deionized water, and air-dried. One hundred microliters of 0.1 N NaOH were added to each well and agitated for 1.5 hours at ambient temperature. Absorbance at 540 nm (A 540) was determined using a microtiter plate reader. Standard collagen amounts were plotted vs. the average A-540 values to produce a standard curve with best fit equation of two variables. One average A-540 value of each conditioned medium sample was selected to substitute for the abscissa variable, to yield the ordinate value, which was corrected for the dilution factor and the volume of material used for testing (10 µl) to give the estimated collagen concentration in the original conditioned medium.

The results in FIG. 7 demonstrate that human Collagen is present at approximately .28 milligram/milliliter of ULSC conditioned media and may readily be concentrated to 1mg/ml.

## Claims

1. A method of using umbilical cord lining stem cells (ULSCs) to produce glycosaminoglycans, the method comprising:
a. Propagating ULSCs in a growth media or conditioning media;
b. Filtering and concentrating the growth media or conditioning media from step a.;
c. Treating the filtered and concentrated growth media or conditioning media from step b. with proteases and nucleases to remove protein and nucleic acid;
d. Treating the treated growth media or conditioning media from step c. with a detergent and an alcohol to remove lipids;
e. Harvesting glycosaminoglycans from the treated growth media or conditioning media from step d.

2. A method according to claim 1 wherein the glycosaminoglycans are selected from one or more of hyaluronic acid and chondroitin sulfate.

3. A method according to claim 2 wherein the hyaluronic acid has a molecular weight of at least 2500 kD.

4. The method of any one of claims 1 to 3 comprising the further step of purifying glycosaminoglycans.

## Patentansprüche

1. Verfahren zum Verwenden von Nabelschnurwandstammzellen (ULSC), um Glykosaminoglykane zu produzieren, wobei das Verfahren Folgendes umfasst:
a. Vermehren von ULSC in einem Wachstumsmedium oder Konditionierungsmedium;
b. Filtern und Konzentrieren des Wachstumsmediums oder Konditionierungsmediums aus Schritt a.;
c. Behandeln des gefilterten und konzentrierten Wachstumsmediums oder Konditionierungsmediums aus Schritt b. mit Proteasen und Nukleasen, um Protein und Nukleinsäure zu entfernen;
d. Behandeln des behandelten Wachstumsmediums oder Konditionierungsmediums aus Schritt c. mit einem Reinigungsmittel und einem Alkohol, um Lipide zu entfernen;
e. Gewinnen von Glykosaminoglykanen aus dem behandelten Wachstumsmedium oder Konditionierungsmedium aus Schritt d.

2. Verfahren gemäß Anspruch 1, wobei die Glykosaminoglykane aus einem oder mehreren von Hyaluronsäure und Chondroitinsulfat ausgewählt sind.

3. Verfahren gemäß Anspruch 2, wobei die Hyaluronsäure ein Molekulargewicht von mindestens 2500 kD aufweist.

4. Verfahren nach einem der Ansprüche 1 bis 3, umfassend den weiteren Schritt eines Reinigens der Glykosaminoglykane.

## Revendications

1. Procédé d'utilisation des cellules souches du revêtement du cordon ombilical (ULSC) pour produire des glycosaminoglycanes, le procédé comprenant :
a. la propagation des ULSC dans un milieu de croissance ou un milieu de conditionnement ;
b. la filtration et la concentration du milieu de croissance ou du milieu de conditionnement de l'étape a ;
c. le traitement du milieu de croissance ou du milieu de conditionnement filtré et concentré de l'étape b. avec des protéases et des nucléases pour éliminer la protéine et l'acide nucléique ;
d. le traitement du milieu de croissance ou du milieu de conditionnement traité de l'étape c. avec un détergent et un alcool pour éliminer les lipides ;
e. la récolte des glycosaminoglycanes du milieu de croissance ou du milieu de conditionnement traité de l'étape d.

2. Procédé selon la revendication 1 dans lequel les glycosaminoglycanes sont choisis parmi un ou plusieurs parmi l'acide hyaluronique et le sulfate de chondroïtine.

3. Procédé selon la revendication 2 dans lequel l'acide hyaluronique a un poids moléculaire d'au moins 2 500 kD.

4. Procédé selon l'une quelconque des revendications 1 à 3 comprenant l'étape supplémentaire de purification des glycosaminoglycanes.
